# EUROPÄISCHE PATENTANMELDUNG

(11) **EP 2 774 555 A1**
(43) Veröffentlichungstag der Anmeldung: **10.09.2014**
(21) Anmeldenummer: 13157855.1
(22) Anmeldetag: 05.03.2013
(51) Int. Cl.: A61B 17/16, A61B 17/17

(54) **Medizinisches Werkzeugsystem**

(71) Anmelder: WALDEMAR LINK GmbH & Co. KG, 22339 Hamburg (DE)
(72) Erfinder: Balzarini, Amos, 22844 Norderstedt (DE)
(74) Vertreter: Raffay & Fleck

(57) **Zusammenfassung**

Mit der Erfindung soll ein medizinisches Werkzeugsystem (1) zum Präparieren einer trapezförmigen Ausnehmung in einer Gelenkfläche eines Knochens mit einem um eine Werkzeugachse (7) zur Rotation antreibbaren Fräswerkzeug (2), welches umfangsseitig wirkende Frässchneiden (6) und an einem bezogen auf die Werkzeugachse (7) axialen Ende wirkende Frässchneiden (6) aufweist, und mit einem an der Gelenkfläche festlegbaren Schablonenteil (3) zum Führen des Fräswerkzeuges (2), wobei ein eine Eindringtiefe des Fräswerkzeuges (2) begrenzender Anschlag (10, 19) vorgesehen ist, derart gestaltet werden, dass mit diesem auch unter räumlich engen Verhältnissen einfach und zuverlässig im Querschnitt trapezförmige Ausnehmungen in einer Gelenkfläche eines Knochens geschaffen werden können.

Zur Lösung dieser Aufgabe wird vorgeschlagen, dass das Schablonenteil (3) ein Basiselement (4) mit einer Schlittenführung (12) und einen in der Schlittenführung (12) zwischen zwei Endanschlägen entlang einer in einer Führungsebene liegenden Führungsbahn bewegbaren Schlitten (5) aufweist, wobei in dem Schlitten (5) ein schräg zu der Führungsebene verlaufender Führungskanal (18) zur quer zu der Werkzeugachse (7) geführten Aufnahme des Fräswerkzeuges (2) in der Weise, dass das Fräswerkzeug (2) frei um die Werkzeugachse (7) drehen kann, gebildet ist

## Beschreibung

Die vorliegende Erfindung betrifft ein medizinisches Werkzeugsystem zum Präparieren einer trapezförmigen Ausnehmung in einer Gelenkfläche eines Knochens mit den Merkmalen des Oberbegriffes des Anspruches 1.

Es ist bereits seit langem üblich, auf dem Gebiet der chirurgischen Orthopädie verschlissene, sklerotische oder durch andere Erkrankungen beschädigte Gelenke ganz oder teilweise durch prothetische Implantate zu ersetzen. So gehören heutzutage insbesondere der Ersatz von Kniegelenken durch entsprechende Implantate wie auch das Setzen künstlicher Hüftgelenke oder Hüftgelenksteile zum Standardoperationsrepertoire der chirurgischen Orthopädie. Dabei haben sich in der Vergangenheit abhängig von der Schwere des Gelenkdefektes ganz unterschiedliche Techniken und Operationsmethoden herausgebildet. Neben Totalersatzprothesen, bei denen z.B. bei einem verschlissenen Kniegelenk sowohl im Bereich des Femurs als auch im Bereich der Tibia die Gelenkflächen und auch angrenzende Knochenabschnitt vollständig durch künstlich gebildete Implantatteile ersetzt werden, sind heutzutage auch Techniken und Konstellationen geläufig, bei denen unter weitest möglichem Erhalt der natürlichen Knochensubstanz nur partielle Ersetzungen von verschlissenen Gelenkflächen vorgenommen werden. So gibt es beispielsweise Implantatsysteme, bei denen in Kniegelenken teilprothetisch lediglich eine der beiden Kondylen der tibiaseitigen Gelenkfläche ersetzt, die andere Kondyle natürlich belassen wird und auch nur auf der der ersetzten, mit dem künstlichen Implantat versorgten Kondylenfläche gegenüberliegender Seite des Femurteils eine künstliche Lauffläche des Gelenkes aufgebracht wird.

Derartige Teilprothesen werden häufig mit leistenförmigen oder anders geformten Fortsätzen im Knochen verankert, z.B. mit sogenannten Finnen. Diese Finnen, die mit einem gewissen Übermaß in eine entsprechende im Knochenmaterial geschaffene Ausnehmung bzw. eine ausgeräumte Nut eingepresst werden, sind dabei häufig im Querschnitt trapezförmig gebildet. Derartige Befestigungsmechanismen können aber nicht nur bei Teilimplantaten verwendet werden, sondern auch für andere am Knochen festzulegende Implantate, auch für Totalersatzprothetik.

Gerade aber dann, wenn schonende Teilersatzprothesen gesetzt werden, wird auch unter der Operation auf einen weitestgehenden Erhalt und eine beschädigungsfreie Belassung der den Gelenkbereich umgebenden Weichteile, insbesondere der Bänder, geachtet. Entsprechend wird eine Trennung des natürlichen Gelenkes so gering wie möglich gehalten, um hier die Belastungen zu reduzieren und den Regenerations- und Heilungsprozess zu befördern, die durch die natürlichen Weichteile gegebene Gelenkstabilität soweit als möglich zu erhalten. Deshalb müssen entsprechende Werkzeugsysteme, mit denen die Strukturen im beschädigten Knochen geschaffen werden müssen, in denen Prothesenteile festgelegt werden, so gebildet sein, dass sie auch in kleinen Spalträumen eingesetzt werden können, dabei jedoch ausreichend präzise wirken.

Während früher es vielfach dem Geschick und handwerklichem Vermögen des Operateurs überlassen wurde, die Gelenkflächen der beteiligten Knochen für den Ersatz verschlissener Gelenkbereiche frei Hand zu präparieren, finden inzwischen vermehrt werkzeugtechnische Hilfsmittel Eingang, um hier dem Operateur das Handwerk zu erleichtern und für eine sichere und präzise Anordnung und Ausbildung der erforderlichen Strukturen zu sorgen.

So sind verschiedentlich Werkzeugsysteme zum Räumen von Knochenmaterial bekannt, bei denen Schablonen mit Fräsen zusammenwirken, um Ausnehmungen vorgegebener Form und Tiefe schaffen zu können.

Ein solches Werkzeugsystem, welches zum Fräsen von Ausnehmungen einer vorgegebenen Kontur verwendet wird, ist in der DE 603 20 485 T2 offenbart. Dort wird in einer plattenartigen Schablone in einem Führungsschlitz geführt ein Fräswerkzeug eingesetzt, welches einen mit einem Radius versehenen Fräskopf aufweist und aufgrund der gegebenen Führung einen kassettenartigen Freiraum ausräumt. In ähnlicher Weise ist ein System mit Schablonenführung und Fräswerkzeug in der DE 195 01 550 A1 offenbart.

In der DE 60 2004 013 383 T2 ist ein System mit einer Schablone und einem darin geführten Fräswerkzeug offenbart für die Präparation der Kondylenflächen einer Knieprothese. In der dort gezeigten Vorrichtung führt die Drehwelle des Fräsers im Wesentlichen senkrecht nach oben, benötigt einen großen Freiraum, so dass während der Operation und der Präparationsphase der Femurteil des Knies weit zurückversetzt werden muss, was mit dem Ziel eines Erhaltes der Weichteile des Gelenkes, insbesondere der Bänder, hier insbesondere der Kreuzbänder, nicht in Übereinstimmung zu bringen ist.

Zudem ist am Markt ein Implantatsystem für einen Teilersatz des Knies mit einer einseitig zu ersetzenden Kondylenfläche und einem ebenfalls einseitig am Femur anzubringenden Gelenkflächenimplantat bekannt, welches von dem US-Unternehmen Stryker Corporation unter der Marke EIUS™ vertrieben wird. Für dieses Implantatsystem ist ein Präparationswerkzeug vorgesehen, in welchem eine trapezförmige Ausnehmung in der zu versorgenden Kondylenfläche der Tibia ausgebildet wird. Hierzu ist ein Schablonenteil mit einem Plattenelement gegeben, welches einen fest mit dem Plattenelement verbundenen, einen rechteckigen Querschnitt aufweisenden Röhrenabschnitt aufweist, der einen Werkzeugtunnel bildet, der in etwa 45° geneigt zu der Ebene der Platte verläuft. Zum Ausbilden der trapezförmigen Ausnehmung wird in diesem Tunnel zunächst eine Bohrschablone eingesetzt, und es werden Schwächungsbohrungen in das Knochenmaterial eingebracht. Anschließend wird der Bohreinsatz entnommen und der so perforierte Knochen durch einen ebenfalls durch den Tunnel eingebrachten Räummeißel fertig geräumt zum Ausbilden der Ausnehmung, in die die Finne des Kondylenimplantates eingebracht wird. Das Werkzeugsystem ist einerseits vielteilig und das damit einhergehende Verfahren zur Ausbildung der Ausnehmung ist umständlich. Denn es müssen hier mit verschiedenen Werkzeugen zunächst die Bohrungen eingebracht und dann die endgültige Räumung der Ausnehmung vorgenommen werden. Diese Vorgehensweise kostet wertvolle Operationszeit und führt zudem dazu, dass im Anschluss der Operation eine Vielzahl von Teilen und Instrumenten zu reinigen und zu desinfizieren sind.

Hier eine Vereinfachung zu geben und ein optimiertes medizinisches Werkzeugsystem zu schaffen, mit dem auch unter räumlich engen Verhältnissen einfach und zuverlässig trapezförmige Ausnehmungen in einer Gelenkfläche eines Knochens geschaffen werden können, ist Aufgabe der Erfindung. Insbesondere soll dieses Werkzeugsystem geeignet sein für das Anbringen einer solchen trapezförmigen Ausnehmung in einer Kondylenfläche des Tibiaanteils des Kniegelenkes, z.B. für die Vorbereitung zum Setzen einer Teilersatzprothese in diesem Bereich.

Diese Aufgabe wird erfindungsgemäß gelöst durch ein medizinisches Werkzeugsystem mit den Merkmalen des Anspruches 1. Vorteilhafte Weiterbildungen dieses neuartigen Werkzeugsystems sind in den abhängigen Ansprüchen 2 bis 9 bezeichnet.

Das neuartige medizinische Werkzeugsystem zum Präparieren einer trapezförmigen Ausnehmung in einer Gelenkfläche eines Knochens nach der Erfindung weist - und dies zunächst in Übereinstimmung mit bekannten vergleichbaren Werkzeugssystemen - ein um eine Werkzeugachse zur Rotation antreibbares Fräswerkzeug auf, welches umfangsseitig wirkende Frässchneiden und zudem Frässchneiden aufweist, die an einem bezogen auf die Werkzeugachse axialen Ende wirken. Es hat ferner - und auch dies in Übereinstimmung mit vorbekannten vergleichbaren Werkzeugsystemen - ein an der Gelenkfläche festlegbares Schablonenteil zum Führen des Fräswerkzeuges, und es ist ein eine Eindringtiefe des Fräswerkzeuges begrenzender Anschlag vorgesehen.

Das Besondere an dem erfindungsgemäßen Werkzeugsystem besteht nun darin, dass das Schablonenteil ein Basiselement mit einer Schlittenführung aufweist und weiterhin in der Schlittenführung zwischen zwei Endanschlägen entlang einer in einer Führungsebene liegenden Führungsbahn bewegbaren Schlitten. Ferner ist erfindungsgemäß in dem Schlitten ein Führungskanal zur Aufnahme des Fräswerkzeuges gebildet. In diesem Führungskanal ist das darin aufgenommene Fräswerkzeug dann quer zu der Werkzeugachse geführt, d.h. es kann sich nicht in einer Richtung quer zu dieser Werkzeugachse relativ zu dem Führungskanal bewegen. In dem Führungskanal kann das Fräswerkzeug sich allerdings frei um die Werkzeugachse drehen und in axialer Richtung bewegt werden.

Das erfindungsgemäße Werkzeugsystem kann z.B. zum Ausbilden einer trapezförmigen Ausnehmung an einer Kondylenfläche des Tibiateils eines Kniegelenkes eingesetzt werden, ist hierfür besonders geeignet. Bei der Präparation der Kondylenfläche zum Setzen eines prothetischen Implantates wird diese zunächst in einem quer zur Längsachse der Tibia geführten Schnitt und einem quer zu diesem Schnitt geführten Trennschnitt derart präpariert, dass dort ein ebenes Plateau entsteht. In dieses ebene Plateau wird dann eine trapezförmige Ausnehmung eingebracht, in die ein Verankerungsfortsatz, typischerweise als Finne bezeichnet, eines entsprechenden Implantates eingepresst werden kann. Hierzu wird nach Präparation des ebenen Plateaus auf die dann entstandene Fläche das Basiselement des Schablonenteils des erfindungsgemäßen Werkzeugsystems aufgesetzt und insbesondere fixiert. Der auf dem Basiselement in der Schlittenführung zwischen den beiden Endanschlägen hin und her bewegbare Schlitten kann nun zwischen diesen Endabschlägen hin und her gleiten, entsprechender Raum steht auch bei minimalinvasiven Operationstechniken zur Verfügung. Durch den Führungskanal in dem Schlitten wird das Fräswerkzeug eingebracht und in Rotation versetzt. Dabei schneidet das Fräswerkzeug in die Knochenoberfläche, dies unter dem voreingestellten Winkel der Schrägstellung des Führungskanals relativ zu der Führungsebene. Durch Hin- und Herbewegen des Schlittens, Vor- und Zurückbewegen des Fräswerkzeuges in dem Führungskanal bis maximal zu dem Anschlag bei gleichzeitiger Rotation des Fräswerkzeuges wird eine entsprechende Ausnehmung frei geräumt. Der Anschlag verhindert, dass das Fräswerkzeug zu tief in den Knochen eindringt und bestimmt die Tiefe der Ausnehmung. Dadurch, dass das Fräswerkzeug sowohl in Umfangsrichtung als auch in Richtung des axialen Endes wirkende Frässchneiden aufweist, räumt es die Ausnehmung an einer durch das axiale Endes des Fräsers gebildeten Flanke, die entsteht, wenn der Schlitten maximal in einer Vorschubrichtung des Fräswerkzeuges in der Schlittenführung bis zum Endanschlag bewegt ist und das Fräswerkzeug bis zum Anschlag in den Führungskanal eingebracht ist, glatt und sauber aus. Die Schrägstellung des Führungskanals auf dem Schlitten ist dabei insbesondere so, dass ein von der Führungsebene wegzeigender Anteil an einem vorderen, d.h. während der Operation proximal anzusetzenden Ende des Schlittens gelegen ist. Hierdurch entsteht eine Art Keilform des Führungskanals auf dem Schlitten, mit der geringeren Höhe in Richtung des distalen Endes des natürlichen Kniegelenkes, so dass dort die benötigte Arbeitshöhe am geringsten ist, die während der Operation typischerweise dorthin verlagerten weiteren Teile des Kniegelenkes, nämlich der Femur mit den Weichteilen, insbesondere den Bändern, den Vorgang nicht stören, entsprechend substanzerhaltend gearbeitet werden kann.

Anders als z.B. bei dem bekannten System des Anbieters Stryker Corporation wird hier mit einem einzigen Werkzeug, nämlich dem Fräswerkzeug, die vollständige Ausräumung der Ausnehmung bewerkstelligt, und es muss nicht eine Vorarbeit mit Knochenbohrern und ein anschließendes Fertigausräumen mit einem Meißelwerkzeug erfolgen.

Mit Vorteil ist, wie gemäß einer Weiterbildung der Erfindung vorgesehen, das Basiselement plattenförmig und eben gebildet. Mit der plattenförmigen und ebenen Gestaltung passt sich das Basiselement besonders gut der typischerweise eben vorbereiteten Fläche nach dem Kondylenschnitt an. Darüber hinaus ist mit einem plattenförmigen Basiselement eine besonders geringe Bauhöhe zu erzielen, was im Hinblick auf die oben bereits geschilderten Ziele eines minimalen Eingriffes in die natürliche Gelenkstruktur von Vorteil ist. Die Stärke des plattenförmigen Basiselementes wird dabei in der Regel derart gewählt werden, dass eine ausreichend stabile und genaue Führung des Schlittens möglich ist, zugleich eine möglichst geringe Stärke eingestellt wird.

Mit Vorteil kann bei dem erfindungsgemäßen Werkzeugsystem die Schlittenführung in dem Basiselement durch einen gerade verlaufenden Längsschlitz gebildet sein, in dem ein Führungsfortsatz des Führungsschlittens aufgenommen ist. Eine solche Schlittenführung ist einerseits technisch einfach zu realisieren und leicht zu bedienen, andererseits ist sie die ideale Führung für das Bilden einer gerade verlaufenden, im Querschnitt trapezförmigen Ausnehmung. Die Trapezform der Ausnehmung entsteht dabei aufgrund der zu einem Untergrund der Ausnehmung und auch zu der nach dem Kondylenschnitt verbleibenden ebenen Oberfläche schräggestellten Stellung der Achse des Fräswerkzeuges. Eine vordere Begrenzungsfläche ist dieser Achsstellung folgend schräg, eine hintere Begrenzungsfläche wird aufgrund der an dem axialen Ende wirkenden Frässchneiden schräg gebildet, dies insbesondere dann, wenn die an dem bezogen auf die Werkzeugachse axialen Ende wirkenden Frässchneiden bei der Fräsbearbeitung eine Fläche fräsen, die im Wesentlichen plan und senkrecht zu der Rotationsachse ist. Der Fräser kann in einem einfachsten Fall eine zylinderförmige Außenkontur aufweisen mit einem freien axialen Ende, welches im Wesentlichen senkrecht zu der Werkzeugachse verläuft und mit Fräskanten bzw. -schneiden entlang des Umfanges und an dem vorderen Ende.

Der Führungskanal ist mit Vorteil gegenüber der Führungsebene um einen Winkel von 30° bis 60° geneigt, insbesondere in einem Winkel von 40° bis 50°, wobei eine Neigung um einen Winkel von 45° besonders bevorzugt ist. Die genannten Winkelbereiche ergeben solche Schrägstellungen des Fräswerkzeuges, die auch unter Berücksichtigung der langen mit einem Fräsantrieb zu verbindenden Fräswelle nicht mehr störend und raumgreifend in den eigentlichen Gelenkbereich eingreifen, d.h. den Bereich, in dem sich der für die Operation beiseite gelegte Partnerknochen des die zu bearbeitende Gelenkfläche aufweisenden Knochens befindet sowie die Weichteile des Gelenkes liegen. Hierin unterscheidet sich die erfindungsgemäße Lösung deutlich von der DE 60 2004 013 383 T2, die dort aufgrund der senkrechten Führung der Fräserwelle einen erheblichen Raumbedarf hat.

Insbesondere bevorzugt wird hier ein Winkel von 45°, da mit diesem eine trapezförmige Ausnehmung geschaffen wird, deren seitliche, schräg verlaufende Begrenzungsflächen beide unter einem Winkel von 45° verlaufen. Die erste folgt der Neigung der Werkzeugachse gegenüber der Führungsebene; die zweite Seitenfläche ist entsprechend um 90° verkippt geneigt, also um 45° gegenüber der Führungsfläche in der anderen Ausrichtung. Dieser Unterschied von 90° ist bedingt durch die Ausgestaltung des Fräswerkzeuges, welches an seinem axialen Ende eine vertikal zur Werkzeugachse verlaufenden Flächenabschnitt herausarbeitenden so wirkenden Frässchneiden.

Gemäß einer weiteren vorteilhaften Weiterbildung der Erfindung kann der Führungskanal in einem rohrförmigen Abschnitt des Schlittens angeordnet sein, der einen oberen Rand aufweist. Das Fräswerkzeug weist bei dieser Weiterbildung einen quer zu der Rotationsachse überstehenden Kragen auf. Der Rand des rohrförmigen Abschnittes und der Kragen bilden dabei zusammen den Anschlag, über den die Eindringtiefe des Fräswerkzeuges in den Führungskanal und damit die Arbeitstiefe im Knochenmaterial beschränkt wird, um so die Tiefe der trapezförmigen Ausnehmung zu bestimmen und exakt einzuhalten.

Für eine einfachere Reinigung und Sterilisation des Schablonenteils können mit Vorteil Schlitten- und Basiselement lösbar miteinander verbunden sein. Dies verhindert insbesondere das Erfordernis der Reinigung bzw. Sterilisation von ansonsten ggf. bestehenden Spalten im Bereich der beweglichen Verbindung.

Um das Schablonenteil an dem zu bearbeitenden Knochen festzulegen und damit die Lage der mit Hilfe des Schablonenteils und dem mit diesem zusammenwirkenden Fräswerkzeug in den Knochen einzubringenden Ausnehmung einzuhalten, kann das Basiselement des Schablonenteils mit Vorteil Pinlöcher aufweisen. Solche Pinlöcher sind Durchtrittsöffnungen, durch die Befestigungsstifte oder Schrauben, sogenannte Steckpins oder Schraubpins in zuvor in den Knochen eingebrachte Bohrlöcher eingesetzt werden können. Derartige Pins sind hinlänglich bekannt und werden in großem Umfang in der orthopädischen Chirurgie zum Festlegen von Hilfsvorrichtungen und Werkzeugführungen eingesetzt.

Für ein einfaches Positionieren beim korrekten Setzen des Schablonenteils kann an dem Basiselement ein Handgriffabschnitt ausgebildet sein.

Aus der vorstehenden Beschreibung wird deutlich, dass mit dem erfindungsgemäßen Werkzeugsystem auf einfache Weise sehr präzise und schnell eine trapezförmige Ausnehmung in eine Gelenkfläche eines Knochen eingebracht werden kann, ohne dass es hierzu des Einsatzes verschiedenster Werkzeuge etwa bedarf. Dies kann aufgrund der Schrägstellung des Führungskanals relativ zu der Führungsebene auch unter räumlich beengten Verhältnissen unternommen werden, wie sie insbesondere dann vorherrschen, wenn die Operation weitgehend substanzerhaltend erfolgen soll, insbesondere unter Erhalt der Weichteile des Gelenkes, z.B. der Kreuzbänder eines zu versorgenden Kniegelenkes.

Weitere Vorteile und Merkmale der Erfindung ergeben sich aus der nachfolgenden Beschreibung eines Ausführungsbeispiels anhand der beigefügten Figuren. Dabei zeigen:
- Fig. 1: in einer dreidimensionalen Explosionsdarstellung ein erfindungsgemäßes medizinisches Werkzeugsystem zum Präparieren einer im Querschnitt trapezförmigen Ausnehmung in einer Gelenkfläche eines Knochens in einem Ausführungsbeispiel;
- Fig. 2: das medizinische Werkzeugsystem aus Fig. 1 in zusammengefügtem Zustand;
- Fig. 3: das Werkzeugsystem aus Fig. 2 in einer Seitenansicht mit in einer hinteren Endstellung befindlichem Schlitten;
- Fig. 4: in einer der Fig. 3 vergleichbaren Ansicht das Werkzeugsystem aus Fig. 1 mit in einer vorderen Endstellung befindlichem Schlitten;
- Fig. 5: in einer Ansicht von unten das medizinische Werkzeugsystem aus Fig. 1 und im Vergleich dazu die Unterseite eines medizinischen Implantatteils mit einer im Querschnitt trapezförmigen Verankerungsfinne;
- Fig. 6: in einer übereinander gelegten Vergleichsdarstellung an der Seite das medizinische Werkzeugsystem aus Fig. 1 und dahinter die im Querschnitt trapezförmige Finne eines Implantatteils, wobei in dieser Figur der Schlitten in seiner hinteren Anschlagstellung befindlich ist;
- Fig. 7: in einer Darstellung vergleichbar der Fig. 6 das medizinische Werkzeugsystem mit dem Schlitten in einer vorderen Anschlagstellung und in Überlagerung wiederum mit der Ansicht der Finne;
- Fig. 8: in einer perspektivischen Darstellung das medizinische Werkzeugsystem im Einsatz zum Präparieren einer im Querschnitt trapezförmigen Ausnehmung auf einem zuvor herauspräparierten Tibiaplateau eines Kniegelenkes;
- Fig. 9: die in Fig. 8 gezeigte Situation und Anordnung aus einem anderen Blickwinkel, hier in einer Seitenansicht.

In den Figuren ist ein mögliches Ausführungsbeispiel eines erfindungsgemäßen Werkzeugsystems gezeigt und wird nachfolgend anhand dieser Figuren näher beschrieben.

Das erfindungsgemäße Werkzeugsystem ist in den Figuren allgemein mit 1 bezeichnet und umfasst ein rotatorisch antreibbares Fräswerkzeug 2 sowie ein Schablonenteil 3. Das Schablonenteil 3 seinerseits ist zweiteilig gebildet mit einem Basiselement 4 und einem an dem Basiselement 4 festlegbaren, in einer Verfahrrichtung zwischen zwei Anschlagpunkten verfahrbaren Schlitten 5.

Das Fräswerkzeug 2 ist in einer axialen Richtung erstreckt gebildet und weist an einem Räumende Frässchneiden 6 auf, die einerseits umfangsseitig wirken, andererseits auch an ihrem vordersten Ende eine Flächenräumwirkung entfalten entlang einer Räumfläche, die im Wesentlichen senkrecht steht zu der Längsachse 7 des Fräswerkzeuges 2. An seinem dem Räumende mit den Frässchneiden 6 gegenüberliegenden Ende weist das Fräswerkzeug 2 einen Kupplungsanschluss 8 auf zur Verbindung mit einem Rotationsantrieb. Ausgehend von dem mit Frässchneiden 6 besetzten Räumende in Richtung des Endes mit dem Kupplungsanschluss 8 erstreckt sich ein Zylinderabschnitt 9 bis hin zu einer ringförmig umlaufenden Verdickung 10, welche in ihrem Durchmesser größer ist als der Durchmesser des Zylinderabschnittes 9.

Das Schablonenteil 3 ist, wie bereits ausgeführt, zweitteilig aufgebaut und besteht aus einem Basiselement 4 und einem an diesem Basiselement 4 lösbar festlegbaren, in festgelegtem Zustand in einer Schlittenführung geführten Schlitten 5. Das Basiselement 4 ist plattenförmig gebildet mit einem Griffabschnitt 11, an welchem dieses im Gebrauch ergriffen und gehalten werden kann. Es weist ferner einen Längsschlitz 12 auf, der durch eine Kreisbohrung 13 mit gegenüber der Schlitzbreite erweiterten Durchmesser an einer Position geöffnet ist. In einem in etwa halbkreisförmig gebildeten Auflageabschnitt 14 ist eine Werkzeugdurchtrittsausnehmung 15 gebildet. Ferner sind an dem Basiselement 4 insgesamt vier Pinlöcher 16 angeordnet.

Der Schlitten 5 weist auf seiner Unterseite einen Führungspin auf, der mit einem kreisförmigen Rückhalteteller 17 endet. Der Durchmesser dieses kreisförmigen Rückhaltetellers 17 ist in etwa dem Durchmesser der Kreisbohrung 13 entsprechend, so dass der Rückhalteteller 17 durch die Kreisbohrung 13 geführt werden kann, um den Führungspin des Schlittens in den Längsschlitz 12 zur Längsführung des Schlittens 5 einzubringen und dort in allen Positionen mit Ausnahme derjenigen Position, in der der Rückhalteteller 17 und die Kreisbohrung 13 exakt fluchten, zu verriegeln.

An dem Schlitten 5 ist ein Führungskanal 18 ausgebildet, der durch eine einen umlaufenden Rand 19 aufweisende Führungsöffnung und einen sich daran anschließenden, eine umlaufende zylinderförmige Wand aufweisenden Kanal gebildet ist. Der Durchmesser des Führungskanals 18 entspricht im Wesentlichen dem Durchmesser des Zylinderabschnittes 9, so dass das Fräswerkzeug 2 mit seinem mit Frässchneiden 6 bestückten Räumende voran in den Führungskanal 18 eingesetzt und darin seitlich sicher geführt rotieren kann. Dabei ist der Führungskanal 18 derart ausgerichtet, dass er schräg zu einer durch den Längsverlauf des Längsschlitzes 12 gebildeten Schlittenführungsachse liegt, insbesondere um einen Winkel von etwa 45°. An einer dem Griffabschnitt 11 abgewandten Vorderseite des Schlittens 5 ist in Verlängerung des Führungskanals 18 ein Schildabschnitt 20 angeordnet, der dazu dient, im Gebrauch des Werkzeugsystems 1 die Frässchneiden 6 in diesem Bereich abzudecken und insbesondere beim Einführen des Fräswerkzeuges 2 mit seinem Räumende voran in den Führungskanal 18 eine nicht gewollte Verletzung von zu erhaltendem umliegenden Gewebe oder Knochenabschnitten zu verhindern.

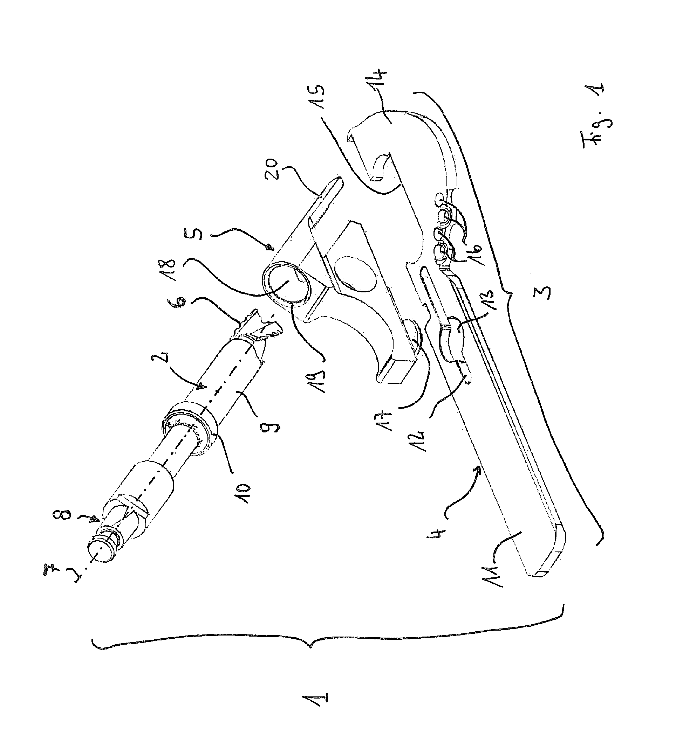

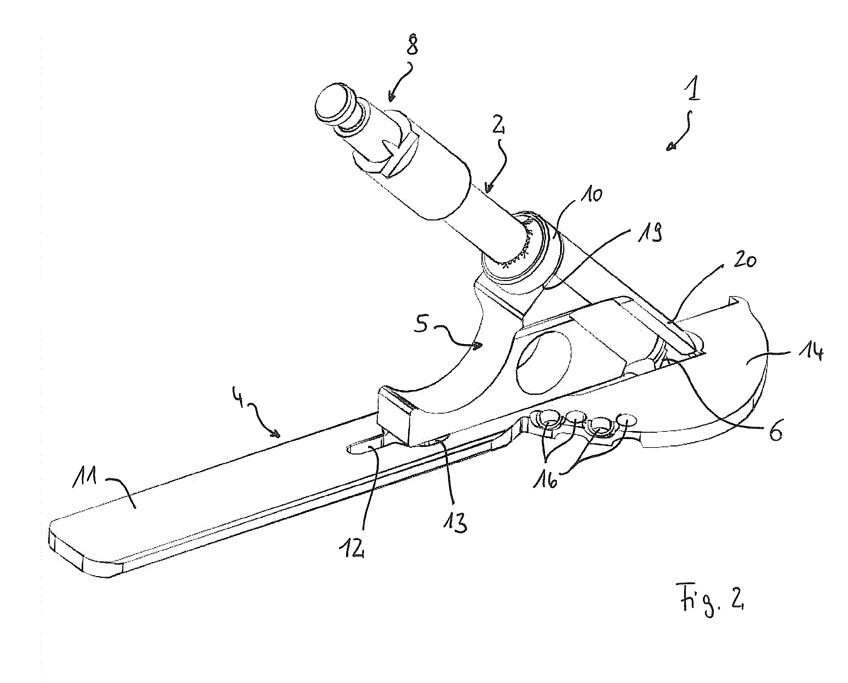

In Fig. 2 ist das in Fig. 1 in Explosionsdarstellung gezeigte Werkzeugsystem 1 in vergleichbarer Perspektive in einem zusammengefügten Zustand dargestellt. Hier ist gut zu erkennen, dass der umlaufende Rand 19 an der Führungsöffnung in den Führungskanal 18 zusammen mit der ringförmigen Verdickung 10 am Fräswerkzeug 2 einen die Eindringtiefe des mit Frässchneiden 6 bestückten Räumendes des Fräswerkzeuges 2 begrenzenden Anschlag bildet.

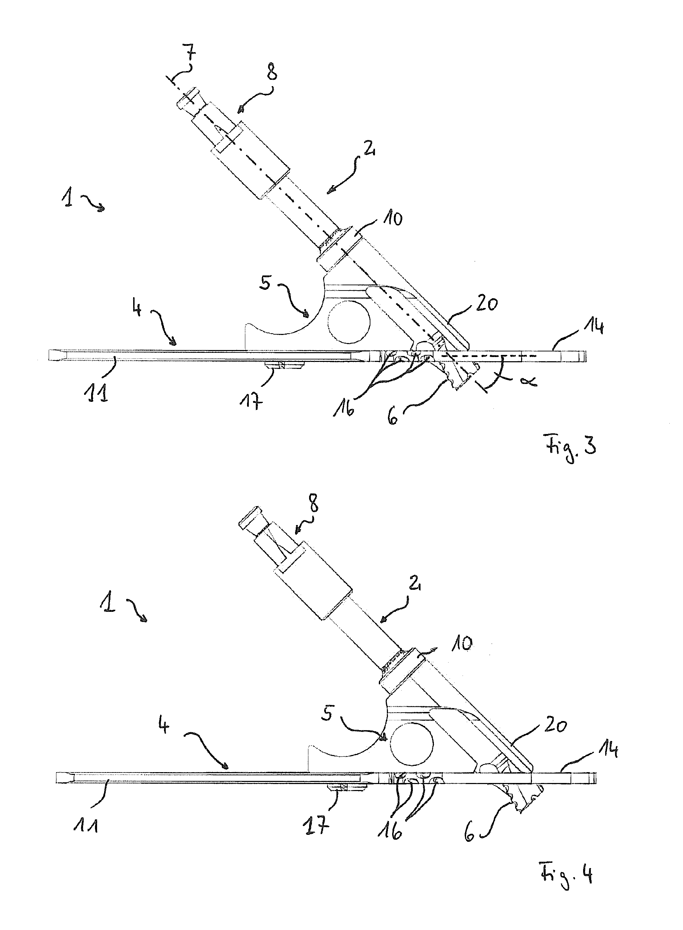

In Fig. 3 ist in einer Seitenansicht gezeigt, wie mit dem Schlitten 5 in einer durch das hintere Ende des Längsschlitzes 12 begrenzten Anschlagsposition befindlich und bei maximal tief in den Führungskanal eingetauchtem Fräswerkzeug 2 dessen Räumende mit den Frässchneiden 6 bis zu einer Arbeitstiefe über eine Ebene des als Platte ausgebildeten Basiselementes 4 vorsteht. Zu erkennen ist hier auch der zwischen der Plattenebene und der Längsachse 7 des Fräswerkzeuges 2 eingeschlossene Winkel α, der hier mit Vorzug 45° beträgt.

In Fig. 4 ist in einer der Fig. 3 vergleichbaren Stellung das medizinische Werkzeugsystem 1 in einer solchen Position gezeigt, in der der Schlitten 5 sich in einer vorderen Anschlagsposition befindet, der durch das vordere Ende des Längsschlitzes 12 bestimmt ist.

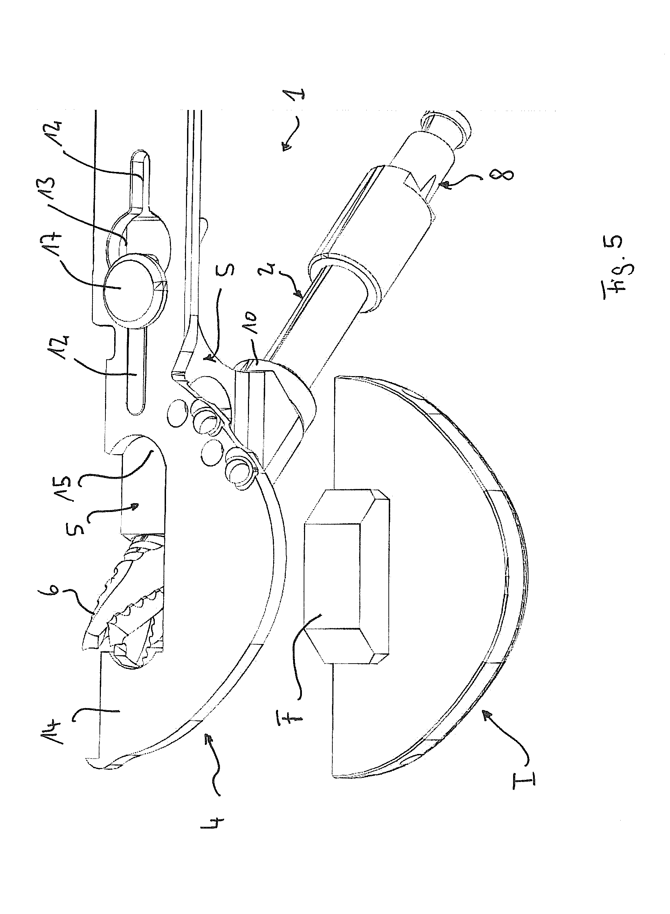

In Fig. 5 ist ein einer oberen Darstellung das Werkzeugsystem 1 in einer Ansicht von unten dargestellt. Dabei ist hier gut zu erkennen, wie das Fräswerkzeug 2 mit dem mit den Frässchneiden 6 bestückten Räumende durch die Werkzeugdurchtrittsausnehmung 15 im Auflageabschnitt 14 des Basiselementes 4 hindurchragt. Auch ist die Führung des Schlittens 5 durch das Zusammenwirken des mit dem Rückhalteteller 17 gehaltenen Pins in dem Längsschlitz 12 gut zu erkennen. In derselben Figur in einer unteren Abbildung ist - ebenfalls in einer Ansicht von unten - ein Implantatteil I dargestellt mit einer Finne F, die im Querschnitt trapezförmig gebildet ist und ein Verankerungselement für das Implantatteil I zum Festlegen desselben an einem Knochen bildet. Das medizinische Werkzeugsystem 1 dient dazu, eine der Form der Finne F im Wesentlichen entsprechende Ausnehmung in einen Knochen zu bringen bzw. aus dem Knochenmaterial auszuräumen.

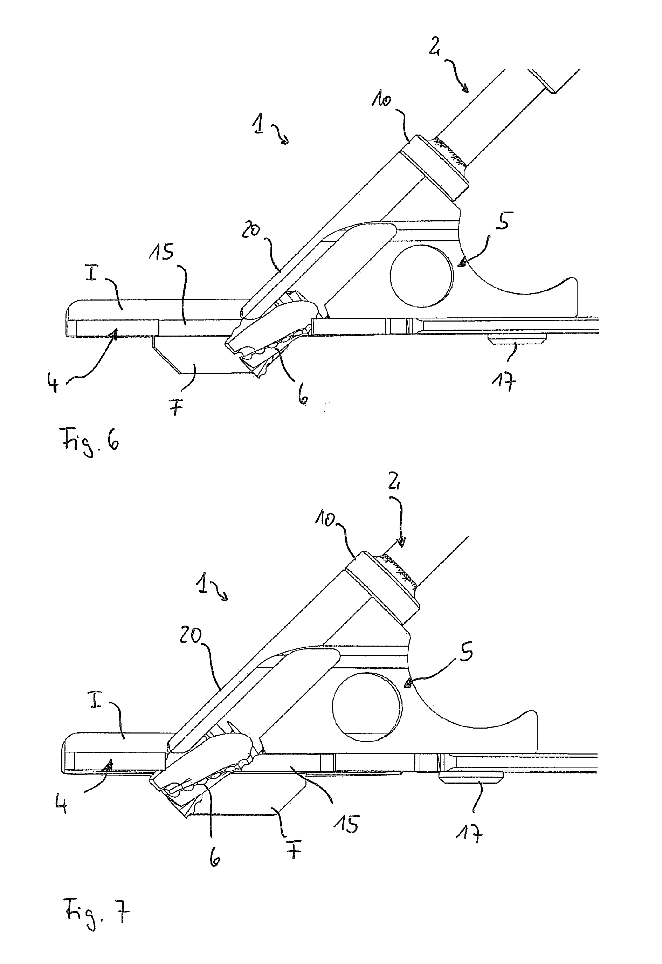

Hier veranschaulichen die Figuren 6 und 7 noch einmal sehr gut, wie dies in der Praxis geschieht. Dort ist in einer Seitenansicht von der den Ansichten gemäß Figuren 3 und 4 gegenüberliegenden Seite, hier also auf die seitlich offene Werkzeugsdurchtrittsausnehmung 15 gesehen, das Werkzeugsystem 1 in einer Teilansicht und Darstellung vor dem Implantatteil I mit der Finne F gezeigt. In Fig. 6 ist das System mit dem Schlitten 5 in maximal zurückgezogener Position dargestellt. Hier ist zu erkennen, wie der in der Figur unten rechts dargestellte Umfangsverlauf des mit Frässchneiden 6 bestückten Räumendes des Fräswerkzeuges 2 dem Verlauf der Finne F in diesem Bereich im Wesentlichen folgt. In der in Fig. 7 dargestellten Position überdeckt die im Wesentlichen senkrecht zur Längsachse des Fräswerkzeuges 2 verlaufende Vorderfläche des Räumendes, an der die Frässchneiden 6 ebenfalls abrasiv wirken, die Flanke der Finne F. So lässt sich sehr gut nachvollziehen, dass durch ein Einführen des Fräswerkzeuges durch den Führungskanal in einer Position, in der der Schlitten sich maximal zurückgezogen befindet, ein Einstich erzeugen lässt, der bis auf den der unteren Kante der Finne F entsprechenden Grund der anzubringenden Ausnehmung reicht. Durch Hin- und Herschieben bzw. Ziehen des Schlittens 5 wird nun eine im Querschnitt trapezförmige und der Außenkontur der Finne F entsprechende Ausnehmung geräumt.

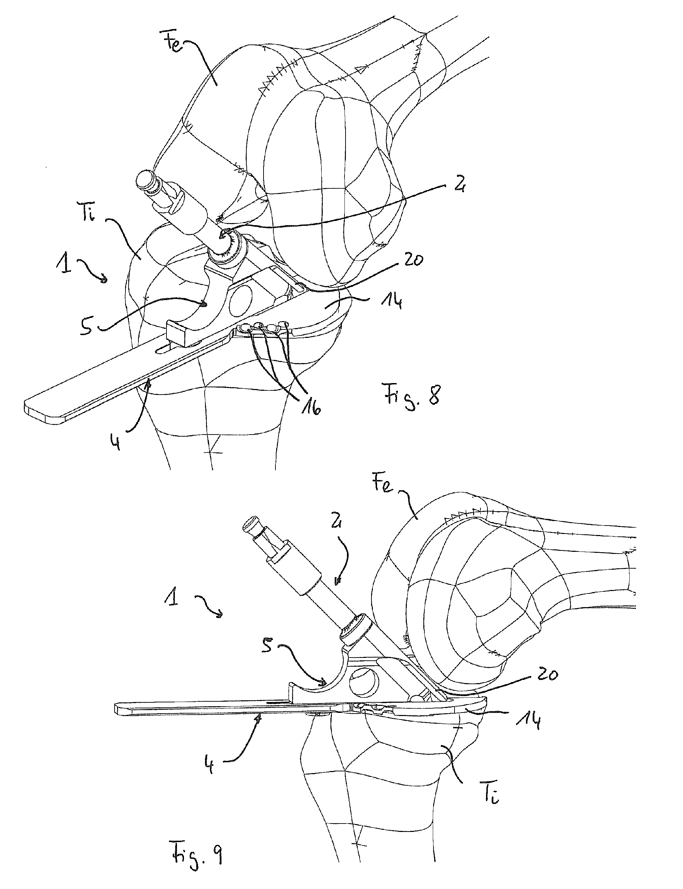

Ein solcher Einsatz in der Praxis ist in den Figuren 8 und 9 aus zwei verschiedenen Ansichten gezeigt. Dort ist gezeigt, wie das hier für das Ausbilden einer entsprechenden Ausnehmung zur Verankerung eines Implantatteils für eine Knieteilersatzprothese vorgesehene Werkzeugsystem 1 verwendet wird. Zu sehen sind hier das distale Ende des Femurs Fe sowie das proximale Ende Tibia Ti. An dem proximalen Ende der Tibia Ti ist einseitig zuvor durch einen Horizontalschnitt ein Plateau geschaffen worden, auf welches das Basisteil 4 des Werkzeugsystems 1 mit seinem Auflageabschnitt 14 aufgesetzt wird. Dabei wird zunächst einmal das Basisteil 4 entsprechend der Position der einzubringenden Vertiefung ausgerichtet und (hier nicht dargestellt) durch Einbringen von einem oder mehreren Haltepins durch eines oder mehrere der Pinlöcher 16 bis in den Knochen in dieser Position fixiert. Anschließend wird der Schlitten 5 aufgebracht und an dem Basiselement 4 in dem Längsschlitz zur Längsführung fixiert, und es wird das Fräswerkzeug 2 durch den Führungskanal in den Schlitten 5 hindurchgeführt und durch Hin- und Herbewegen des Schlittens 5 mit bis zum Anschlag eingeführtem Fräswerkzeug 2 die im Querschnitt trapezförmige Ausnehmung präzise aufgebracht. In den Figuren 8 und 9 ist insbesondere auch sehr gut zu erkennen, dass aufgrund der winkeligen Stellung des Führungskanals das Fräswerkzeug 2 mit seiner Achse sicher aus dem Bereich herausragt, in welchem zwischen Tibia Ti und Femur Fe ein besonders kleiner Spalt belassen ist. Dadurch muss für die Operation der Femur Fe nicht so weit von der Tibia Ti getrennt werden wie sonst üblich, so dass eine Weichteil schonende Operation möglich ist, insbesondere unter Erhalt der natürlichen Bänder, insbesondere auch der Kreuzbänder. Durch die Keilförmigkeit des Schlittens in seinem dem Auflageabschnitt 14 zugewandten Bereich ist hier der Raumbedarf besonders gering. Zu erkennen ist auch, dass der Schildabschnitt 20 hier verhindert, dass bei einem Arbeiten mit dem Fräswerkzeug 2 versehentlich der Femur Fe oder angrenzende Weichteile beschädigt werden, da der Schildabschnitt 20 dort das Räumende des Fräswerkzeuges 2 mit den Frässchneiden sicher abschirmt.

Das gezeigte Ausführungsbeispiel ist nicht beschränkend zu verstehen, sondern zeigt lediglich eine von mannigfaltig möglichen Verwirklichungen des erfindungsgemäßen medizinischen Werkzeugsystems.

### Bezugszeichenliste

- 1: Medizinisches Werkzeugsystem
- 2: Fräswerkzeug
- 3: Schablonenteil
- 4: Basiselement
- 5: Schlitten
- 6: Frässchneiden
- 7: Längsachse
- 8: Kupplungsanschluss
- 9: Zylinderabschnitt
- 10: ringförmige Verdickung
- 11: Griffabschnitt
- 12: Längsschlitz
- 13: Kreisbohrung
- 14: Auflagabschnitt
- 15: Werkzeugdurchtrittsausnehmung
- 16: Pinloch
- 17: Rückhalteteller
- 18: Führungskanal
- 19: umlaufender Rand
- 20: Schildabschnitt

- α: Winkel

- F: Finne
- Fe: Femur
- I: Implantatteil
- Ti: Tibia

## Patentansprüche

1. Medizinisches Werkzeugsystem zum Präparieren einer trapezförmigen Ausnehmung in einer Gelenkfläche eines Knochens (Ti) mit einem um eine Werkzeugachse (7) zur Rotation antreibbaren Fräswerkzeug (2), welches umfangsseitig wirkende Frässchneiden (6) und an einem bezogen auf die Werkzeugachse (7) axialen Ende wirkende Frässchneiden (6) aufweist, und mit einem an der Gelenkfläche festlegbaren Schablonenteil (3) zum Führen des Fräswerkzeuges (2), wobei ein eine Eindringtiefe des Fräswerkzeuges (2) begrenzender Anschlag (10, 19) vorgesehen ist, **dadurch gekennzeichnet, dass** das Schablonenteil (3) ein Basiselement (4) mit einer Schlittenführung (12) und einen in der Schlittenführung (12) zwischen zwei Endanschlägen entlang einer in einer Führungsebene liegenden Führungsbahn bewegbaren Schlitten (5) aufweist, wobei in dem Schlitten (5) ein schräg zu der Führungsebene verlaufender Führungskanal (18) zur quer zu der Werkzeugachse (7) geführten Aufnahme des Fräswerkzeuges (2) in der Weise, dass das Fräswerkzeug (2) frei um die Werkzeugachse (7) drehen kann, gebildet ist.

2. Werkzeugsystem nach Anspruch 1, **dadurch gekennzeichnet, dass** das Basiselement (4) plattenförmig und eben gebildet ist.

3. Werkzeugsystem nach einem der vorhergehenden Ansprüche, **dadurch gekennzeichnet, dass** die Schlittenführung (12) in dem Basiselement (4) durch einen gerade verlaufenden Längsschlitz gebildet ist, in dem ein Führungsfortsatz des Führungsschlittens (5) aufgenommen ist.

4. Werkzeugsystem nach einem der vorhergehenden Ansprüche, **dadurch gekennzeichnet, dass** der Führungskanal (18) gegenüber der Führungsebene um einen Winkel von 30° bis 60° geneigt ist, insbesondere um einen Winkel von 40° bis 50°, besonders bevorzugt um einen Winkel von 45°.

5. Werkzeugsystem nach einem der vorhergehenden Ansprüche, **dadurch gekennzeichnet, dass** die an dem bezogen auf die Werkzeugachse (7) axialen Ende wirkenden Frässchneiden (6) bei der Fräsbearbeitung eine Fläche fräsen, die im Wesentlichen plan und senkrecht zu der Werkzeugachse (7) ist.

6. Werkzeugsystem nach einem der vorhergehenden Ansprüche, **dadurch gekennzeichnet, dass** der Führungskanal (18) in einem rohförmigen Abschnitt des Schlittens (5) angeordnet ist, der oberen Rand (19) aufweist, und dass das Fräswerkzeug (2) einen quer zu der Werkzeugachse überstehenden Kragen (10) aufweist, wobei der Rand (19) und der Kragen (10) zusammen den Anschlag (10, 19) bilden.

7. Werkzeugsystem nach einem der vorhergehenden Ansprüche, **dadurch gekennzeichnet, dass** Schlitten (5) und Basiselement (4) des Schablonenteils (3) lösbar miteinander verbunden sind.

8. Werkzeugsystem nach einem der vorhergehenden Ansprüche, **gekennzeichnet durch** Pinlöcher (16) in dem Basiselement (4).

9. Werkzeugsystem nach einem der vorhergehenden Ansprüche, **gekennzeichnet durch** einen Handgriffabschnitt (11) am Basiselement (4).
